# EUROPÄISCHE PATENTSCHRIFT

(11) **EP 0 684 027 B1**
(45) Veröffentlichungstag und Bekanntmachung des Hinweises auf die Patenterteilung: **18.08.1999**
(21) Anmeldenummer: 95106665.3
(22) Anmeldetag: 03.05.1995
(51) Int. Cl.: A61F 5/02

(54) **Bandage, insbesondere zur Stabilisierung der Iliosakralgelenke**
Brace, in particular for stabilizing the iliosacral joint
Ceinture notamment pour la stabilisation de l'articulation iliosacre

(30) Priorität: 25.05.1994 DE 4418201
(43) Veröffentlichungstag der Anmeldung: 29.11.1995
(73) Patentinhaber: BIEDERMANN MOTECH GmbH, 78054 Villingen-Schwenningen (DE)
(72) Erfinder: Biedermann, Lutz, D-78048 Villingen (DE); Harms, Jürgen, Prof. Dr., D-76133 Karlsruhe (DE)
(74) Vertreter: Prüfer, Lutz H., Dipl.-Phys.

(56) Entgegenhaltungen:
- EP-A- 0 410 904
- EP-A- 0 507 513
- WO-A-93/18724
- WO-A-93/21869
- DE-A- 3 613 235
- US-A- 4 475 543
- US-A- 5 147 261

## Beschreibung

Die Erfindung betrifft eine Bandage, insbesondere zur Stabilisierung der Iliosakralgelenke nach dem Oberbegriff des Patentanspruches 1.

Aus der DE 36 13 235 A1 ist eine Rückenstützbandage bekannt, die ein Rückenteil, seitliche Bauchzüge und einen Verschluß zum Verbinden der Bauchzüge aufweist, wobei das Rückenteil aus einem vielzügigen Material und die Bauchzüge aus einem im wesentlichen einzügigen Material gebildet sind.

Eine derartige Rückenstützbandage gewährleistet durch die Bildung der Bauchzüge aus einem Material mit einer Dehnungsrichtung, die sich von dem Rückenteil in Richtung der bauchseitigen Verschlußteile hin erstreckt, eine Abstützung der Weichteile im Bauchbereich.

Zur Linderung von Beschwerden, die von den Iliosakralgelenken verursacht werden, ist jedoch eine Rückenstützbandage der oben beschriebenen Art weniger geeignet, da die Iliosakralgelenke durch diese nicht ausreichend stabilisiert werden.

Aufgabe der Erfindung ist es daher, eine Bandage der eingangs beschriebenen Art zu schaffen, die so ausgebildet ist, daß sie eine gute Stabilisierung der Iliosakralgelenke ermöglicht.

Diese Aufgabe wird durch eine Bandage mit den Merkmalen des Patentanspruches 1 gelöst.

Die Bandage weist den Vorteil auf, daß einerseits die Wirbelsäule durch die innere Bandage gestützt wird und andererseits die Darmbeinschaufeln durch die äußere Bandage nach hinten gezogen und in dieser Stellung gegen das Kreuzbein fixiert werden. Dadurch werden die Iliosakralgelenke stabilisiert.

Weitere Merkmale und Zweckmäßigkeiten der Erfindung ergeben sich aus der Beschreibung eines Ausführungsbeispieles anhand der Figuren.

Von den Figuren zeigen:
- Fig. 1:: eine Draufsicht auf die Rückseite der Bandage und
- Fig. 2:: einen Schnitt entlang der Linie II/II in Fig. 1.

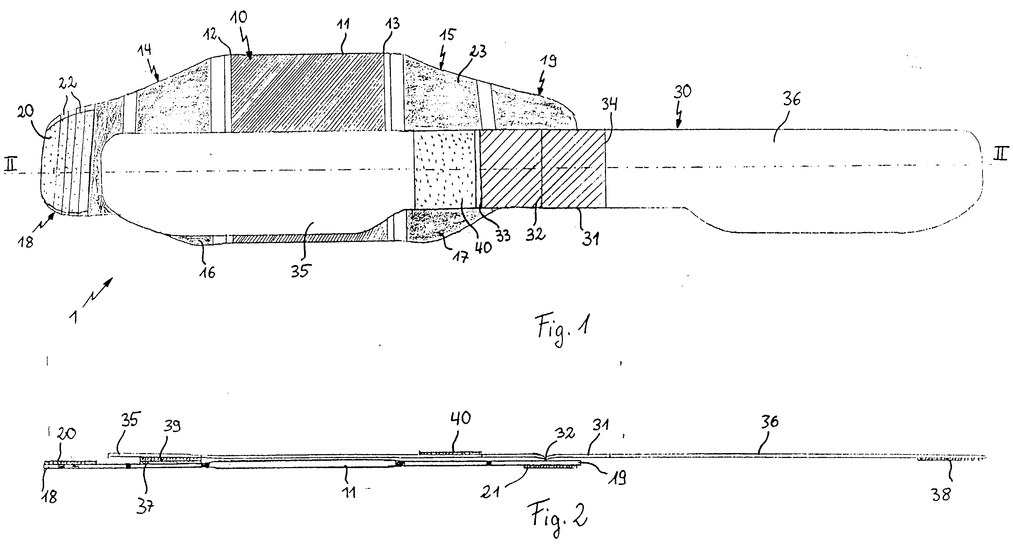

Wie aus Fig. 2 ersichtlich ist, weist die Bandage 1 eine innere Bandage 10 und eine äußere Bandage 30 auf. Die innere Bandage besteht aus einem Rückenteil 11, dessen Breite so gewählt ist, daß das Rückenteil 11 beim Anlegen der Bandage den Rücken im Bereich der Lendenwirbel bedeckt.

Das Rückenteil 11 ist aus einem vielzügigen Material gebildet, welches wenigstens in Richtung quer zur Wirbelsäule und in Richtung der Wirbelsäule dehnbar ist. Ein Beispiel für ein derartiges Material ist ein unter der Handelsbezeichnung Neopren hergestellter Synthesekautschuk.

An den beiden Seitenrändern 12 und 13 des Rückenteiles 11 schließen sich erste und zweite Bauchzüge 14, 15 an, die mit dem Rückenteil jeweils ausreichend fest vernäht sind. Die Bauchzüge 14, 15 weisen unmittelbar an das Rückenteil 11 angrenzende sich über die Seiten erstreckende erste und zweite Seitenabschnitte 16, 17 und daran anschließende sich über den Bauch erstreckende erste und zweite Bauchabschnitte 18, 19 auf. Die durch die Bauchabschnitte 18, 19 gebildeten freien Enden der inneren Bandage 10 weisen jeweils zusammenwirkende Teile 20, 21 eines Klettverschlusses auf, wie aus Fig. 2 ersichtlich ist.

Die beiden Bauchzüge 14, 15 sind aus einem einzügigen Material gebildet, dessen Dehnungsrichtung sich von dem Rückenteil 11 zu den freien Enden der Bauchzüge bzw. zu den Verschlußteilen 20, 21 erstreckt. Die Zugrichtung erstreckt sich also im wesentlichen senkrecht zu der Richtung die der Wirbelsäule entspricht, wenn die Bandage angelegt ist.

An dem freien Ende des ersten Bauchabschnitts 18 sind dünne Kunststoffplatten bzw. Stege 22, die bei geschlossener Bandage im wesentlichen parallel zur Wirbelsäule verlaufen vorgesehen, die als Abdominalverstärkungen wirken.

Auf der Außenseite 23 der inneren Bandage 10 ist die äußere Bandage 30 vorgesehen. Diese umfaßt einen zentralen Abschnitt 31, der, wie am besten aus Fig. 1 ersichtlich ist, schmaler als die Bauchabschnitte 18,19 der inneren Bandage ist und der über eine Naht 32 an dem zweiten Bauchabschnitt 19 festgenäht ist. Die Naht 32 erstreckt sich quer zu der durch den aus den Teilen 20 und 21 gebildeten Verschluß bestimmten Zugrichtung. Der zentrale Abschnitt 31 ist aus einem nichtelastischen Material, beispielsweise aus einem nichtelastischen Stoff, gebildet.

An den beiden Seitenrändern 33 und 34 des zentralen Abschnittes 31 schließen sich bandförmige erste und zweite Seitenabschnitte 35, 36 an, welche an ihren dem zentralen Abschnitt 31 abgewandten freien Enden an ihrer der inneren Bandage zugewandten Seite Teile von weiteren Klettverschlüssen 37, 38 aufweisen, wie am besten aus Fig. 2 ersichtlich ist. Die Seitenabschnitte 35 und 36 haben jeweils eine Länge, die in etwa der Länge der inneren Bandage zwischen dem Beginn des ersten Bauchabschnittes 18 und dem Beginn des zweiten Bauchabschnittes 19 entspricht.

Wie aus Fig. 2 ersichtlich ist, weist der dem die Naht 32 enthaltenden zweiten Bauchabschnitt 19 gegenüberliegende erste Bauchabschnitt 14 an seiner Außenseite im Bereich seines Seitenabschnitts 16 ein Gegenstück 39 zu dem Klettverschlußteil 37 auf. Der sich in Richtung von dem die Naht 32 enthaltenden zweiten Bauchabschnitt 19 zum Rückenteil 11 der inneren Bandage 10 erstreckende erste Seitenabschnitt 35 der äußeren Bandage 30 weist an seiner der inneren Bandage abgewandten Außenseite angrenzend an den zentralen Abschnitt 31 das Gegenstück 40 zu dem Klettverschlußteil 38 des zweiten Seitenabschnittes 36 auf.

Die Seitenabschnitte 35 und 36 der äußeren Bandage 30 sind wiederum aus einem einzügigen Material gebildet von der Art, wie der der Bauchzüge 14 und 15.

Im Gebrauch gelangen die Klettverschlußteile 20, 21 der Bauchabschnitte 18, 19 der inneren Bandage 10 miteinander auf der Bauchseite in Eingriff. Die innere Bandage weist eine gute Formschlüssigkeit zwischen dem Rückenteil 11 und den Lendenwirbelsegmenten auf. Die einzügig ausgebildeten Bauchzüge 14, 15 bewirken einen einwandfreien Sitz der gesamten inneren Bandage 10 und eine genau definierte Kraft und eine Richtung des Zuges auf die Weichteile des Bauches. Die innere Bandage 10 dient außerdem als Träger für die äußere Bandage 30.

Bei der Befestigung der äußeren Bandage wird zunächst der erste Seitenabschnitt 35 gespannt und der Klettverschlußteil 37 der inneren Bandage mit dem Klettverschlußteil 39 des dem Rückenteil 11 zugewandten Seitenabschnitts 35 der äußeren Bandage in Eingriff gebracht. Gleichzeitig ist der erste Seitenabschnitt 35 der äußeren Bandage um das Rückenteil 11 und die Seitenabschnitte 16, 17 der Bauchzüge der inneren Bandage gespannt. Der zweite Seitenabschnitt 36 der äußeren Bandage ist erst um den Bauch und dann um den Rücken herumgespannt, so daß sein Klettverschlußteil 38 mit dem Klettverschlußteil 40 des seitlichen Bauchabschnitts 16 der inneren Bandage in Eingriff gelangt.

Bei angelegter Bandage 1 befindet sich der aus nichtelastischem Material gebildete zentrale Abschnitt 31 der äußeren Bandage 30 über dem Bauch. Durch das einzügig ausgebildete Material der Seitenabschnitte 35, 36 der äußeren Bandage entsteht somit ein Zug an den Rändern 33, 34 des zentralen Abschnitts 31, der bewirkt, daß die Darmbeinschaufeln nach hinten, in Richtung des Rückenteils 11 gedrückt werden. Die Darmbeinschaufeln sind dann in dieser Stellung durch die Bandage fixiert. Dadurch werden die Iliosakralgelenke gegen das Kreuzbein stabilisiert.

## Patentansprüche

1. Bandage, insbesondere zur Stabilisierung der Iliosakralgelenke, mit einer inneren Bandage (10) mit einem Rückenteil (11) und seitlichen Bauchzügen (14, 15) und einem Verschluß (20, 21) zum Verbinden der Bauchzüge, wobei das Rückenteil aus einem vielzügigen Material und die Bauchzüge aus einem im wesentlichen einzügigen Material gebildet sind, und mit einer äußeren Bandage (30) mit einem zentralen Abschnitt (31) und zwei jeweils seitlich an den zentralen Abschnitt (31) angrenzenden elastischen Seitenabschnitten (35, 36)
dadurch gekennzeichnet, daß der zentrale Abschnitt (31) der äußeren Bandage (30) an einem der Bauchzüge (14, 15) der inneren Bandage (10) im Bauchbereich befestigt ist, und aus einem nicht-elastischen Material besteht.

2. Bandage nach Anspruch 1,
dadurch gekennzeichnet, daß die Seitenabschnitte (35, 36) aus dem im wesentlichen einzügigen Material gebildet sind.

3. Bandage nach Anspruch 2,
dadurch gekennzeichnet, daß sich die Dehnungsrichtung des die Seitenabschnitte (35, 36) bildenden Materials von dem zentralen Abschnitt (31) zu dem Rückenteil (11) der inneren Bandage erstreckt.

4. Bandage nach einem der Ansprüche 2 bis 3,
dadurch gekennzeichnet, daß eine Fixierungsvorrichtung (37, 38, 39, 40) zum Fixieren der Seitenabschnitte (35, 36) der äußeren Bandage vorgesehen ist.

5. Bandage nach Anspruch 4,
dadurch gekennzeichnet, daß die Fixierungsvorrichtung (37, 38, 39, 40) jeweils an den Seitenabschnitten (35, 36) angebrachte Verschlußteile (37, 38), die jeweils mit einem an der inneren Bandage bzw. an dem jeweiligen anderen Seitenabschnitt vorgesehenen Gegenstück (39, 40) in Eingriff gelangen, aufweist.

6. Bandage nach Anspruch 5,
dadurch gekennzeichnet, daß die Verschlußteile (37, 38) und ihre Gegenstücke (39, 40) Klettverschlüsse sind.

7. Bandage nach einem der Ansprüche 1 bis 6,
dadurch gekennzeichnet, daß die Bauchzüge (14, 15) an ihren die Verschlußteile (20, 21) aufnehmenden Seiten Abdominalverstärkungen (22) aufweisen.

8. Bandage nach Anspruch 7,
dadurch gekennzeichnet, daß die Abdominalverstärkungen (22) als Kunststoffplatten bzw. -stege ausgebildet sind.

## Claims

1. Brace, in particular for stabilising the iliosacral joints, comprising an interior brace (10) having a dorsal part (11) and lateral abdominal ties (14, 15) and a closure (20, 21) for connecting said abdominal ties, wherein said dorsal part is formed by a multi-tension material and said abdominal ties are constituted by an essentially single-tension material, and comprising an exterior brace (30) with a central section (31) and two elastic lateral parts (35, 36) whereof each is adjacent to the side of said central section (31),
**characterised** in that said central section (31) of said exterior brace (30) is fastened at one of said abdominal ties (14, 15) of said interior brace (10) in the abdominal zone, and consists of a non-elastic material.

2. Brace according to Claim 1,
**characterised** in that said elastic lateral parts (35, 36) are made of a substantially single-tension material.

3. Brace according to Claim 2,
**characterised** in that the direction of extension of the material forming said lateral parts (35, 36) extends from said central section (31) towards said dorsal part (11) of said interior brace.

4. Brace according to any of the Claims 2 to 3,
**characterised** in that a fixing means (37, 38, 39, 40) is provided for fixing said lateral parts (35, 36) of said exterior brace.

5. Brace according to Claim 4,
**characterised** in that said fixing means (37, 38, 39, 40) comprises closing elements (37, 38) respectively attached at said lateral parts (35, 36), whereof each comes into engagement in a counterpart (39, 40) arranged at said interior brace or at the other lateral part, respectively.

6. Brace according to Claim 5,
**characterised** in that said closing elements (37, 38) and their counterparts (39, 40) are Velcro fasteners.

7. Brace according to any of the Claims 1 to 6,
**characterised** in that said lateral abdominal ties (14, 15) comprise abdominal reinforcements (22) at their sides receiving said closing elements (20, 21).

8. Brace according to Claim 7,
**characterised** in that said abdominal reinforcements (22) are designed in the form of plastic plates or webs.

## Revendications

1. Ceinture, notamment pour la stabilisation des articulations iliosacrales, comprenant un bandage intérieur (10) à une partie dorsale (11) et des liens de contrainte latéraux abdominaux (14, 15) et une fermeture (20, 21) à relier lesdits liens de contrainte abdominaux, dans lequel ladite partie dorsale est formée d'un matériau à contrainte multiple et lesdits liens de contrainte abdominaux sont formés d'un matériau essentiellement à simple contrainte, et comprenant un bandage extérieur (30) à un tronçon central (31) et deux parties élastiques latérales (35, 36) dont chacune est contigue au côté dudit tronçon central (31),
**caractérisé** en ce que ledit tronçon central (31) dudit bandage extérieur (30) est fixé à un desdits liens de contrainte abdominaux (14, 15) dudit bandage intérieur (10) dans la zone abdominale, et consiste en un matériau non-élastique.

2. Bandage selon la revendication 1,
**caractérisé** en ce que lesdites parties élastiques latérales (35, 36) sont formées d'un matériau essentiellement à simple contrainte.

3. Bandage selon la revendication 2,
**caractérisé** en ce que la direction d'extension du matériau formant lesdites parties latérales (35, 36) s'étend à partir dudit tronçon central (31) vers ladite partie dorsale (11) dudit bandage intérieur.

4. Bandage selon une quelconque des revendications 2 à 3,
**caractérisé** en ce qu'un moyen fixateur (37, 38, 39, 40) est disposé pour la fixation desdites parties latérales (35, 36) dudit bandage extérieur.

5. Bandage selon la revendication 4,
**caractérisé** en ce que ledit moyen fixateur (37, 38, 39, 40) comprend des éléments de fermeture (37, 38) respectivement montés auxdites parties latérales (35, 36), dont chacun entre en prise dans un pendant (39, 40) disposé audit bandage intérieur ou respectivement à l'autre partie latérale respective.

6. Bandage selon la revendication 5,
**caractérisé** en ce que lesdits éléments de fermeture (37, 38) et leurs pendants (39, 40) sont des fermetures du type Velcro.

7. Bandage selon une quelconque des revendications 1 à 6,
**caractérisé** en ce que lesdits liens de contrainte latéraux abdominaux (14, 15) comprennent des renforts abdominaux (22) à leurs côtés qui reçoivent lesdits éléments de fermeture (20, 21).

8. Bandage selon la revendication 7,
**caractérisé** en ce que lesdits renforts abdominaux (22) sont configurés sous forme de plaques ou traverses plastiques.
